# EUROPEAN PATENT SPECIFICATION

(11) **EP 3 519 809 B1**
(45) Date of publication and mention of the grant of the patent: **04.11.2026**
(21) Application number: 17857193.1
(22) Date of filing: 14.09.2017
(51) Int. Cl.: G01N 33/00, B01D 46/00

(54) **GAS SENSOR MODULE WITH FIELD REPLACEABLE, INGRESS PROTECTED, SENSOR FILTER**
GASSENSORMODUL MIT VOR ORT AUSTAUSCHBAREM EINTRITTSGESCHÜTZTEM SENSORFILTER
MODULE DE CAPTEUR DE GAZ COMPRENANT UN FILTRE DE CAPTEUR PROTÉGÉ CONTRE TOUTE PÉNÉTRATION, REMPLAÇABLE SUR LE TERRAIN

(30) Priority: 29.09.2016 US 201662401440 P; 03.03.2017 US 201715449359
(43) Date of publication of application: 07.08.2019
(73) Proprietor: Rosemount Inc., Shakopee, MN 55379 (US)
(72) Inventor: MORRIS, Mark, Cokato Minnesota 55321 (US); GINDELE, Greg, Maple Lake Minnesota 55358 (US); CHHUOY, Hun, Savage Minnesota 55378 (US)
(74) Representative: Meissner Bolte Partnerschaft mbB
(86) International application number: PCT/US2017/051443
(87) International publication number: WO 2018/063806

(56) References cited:
- EP-A2- 1 197 753
- WO-A1-2015/038217
- WO-A1-2016/030735
- WO-A1-2016/030735
- DE-U1- 202008 018 217
- US-A- 5 879 527
- US-A1- 2011 138 881
- US-A1- 2013 145 845
- US-B1- 8 852 513

## Description

### BACKGROUND

The process industry often employs gas sensors in order to detect the presence of a particular gas, often as part of a safety system. This is important as many gases may be harmful to human health and/or the environment. Industrial gas sensors are normally mounted near the process area of a plant or control room, or an area to be protected. Generally, industrial gas sensors are installed at fixed locations and to communicate with monitoring systems. EP 1 197 753 is considered to be the closest prior art and describes a gas detection device according to the preamble of claim 1. Further related prior art can be seen from: WO 2016/030735 and US 5,879,527.

### SUMMARY

The invention is defined by the gas detection device according to claim 1 which includes a gas sensor module, configured to detect a gas, and a filter assembly. The gas sensor module includes a gas sensor disposed within a gas sensor housing and circuitry coupled to the gas sensor housing. The circuitry is configured to provide an indication of the gas. The filter assembly is configured to couple to the gas sensor housing and protect the gas sensor from particulates. At least one latching feature is provided and is configured to couple to the gas sensor module, the at least one latching feature comprising an inverted U-shaped body having a protrusion configured to be received by a receiving portion on the gas sensor module. The filter assembly comprises an alignment mechanism configured to align with a corresponding alignment portion on the gas sensor housing, and wherein the alignment mechanism is a groove on the filter assembly configured to receive the corresponding alignment portion on the gas sensor housing. A related method is given in claim 8.

### BRIEF DESCRIPTION OF THE DRAWINGS

FIG. 1 is a cross sectional view of a gas sensor module coupled to a filter assembly in accordance with an embodiment of the present invention.
FIG. 2 is a block diagram of a gas sensor module in accordance with an embodiment of the present invention.
FIG. 3 is a diagrammatic view of a filter assembly that is field-replaceable in accordance with an embodiment of the present invention.
FIG. 4A and 4B are diagrammatic views of a filter assembly aligned with a gas sensor module in accordance with an embodiment of the present invention.
FIG. 5 is a flow diagram illustrating an operation of coupling a filter assembly to a gas sensor module in accordance with an embodiment of the present invention.

### DETAILED DESCRIPTION OF ILLUSTRATIVE EMBODIMENTS

Gas sensors may be used to detect combustible, flammable and toxic gases. Gas sensors may include infrared point sensors, ultrasonic sensors, electrochemical gas sensors, and semiconductor sensors. To work properly, gas sensors must not become compromised by moisture, dust, or other types of debris. If compromised, gas sensors may not be able to accurately sense gases, and, over time, may even become damaged leading to their eventual replacement.

Often, gas sensors are protected using a barrier to shield out unwanted particulates. However, to install the barrier, a known protocol is often followed requiring a number of different tools. Additionally, these protocols often take extended periods of time to complete, and may lead to a damaged gas sensor if not followed correctly. For example, upon installing the barrier using a threaded method, damage to a sealing mechanism may occur during installation.

Contrary to conventional systems, embodiments described herein provide a replaceable, ingress protection (IP) rated filter assembly that allows for an exclusive passage of gas to a gas sensor while protecting the gas sensor from moisture and debris. The Ingress Protection ratings, as defined in the international standard IEC 60529, classify the degree of protection provided against the intrusion of solid objects (including body parts), dust, accidental contact, and water in electrical enclosures. One example Ingress Protection rating is the IP66 rating which, per the standard, ensures complete protection against contact and dust, along with offering protection against moisture up to a specified degree which, per the standard, includes protection against water from powerful jets such that water does not enter the enclosure in harmful quantities. Additionally, while protecting the gas sensor from moisture and debris per an ingress protection standard, the filter assembly allows the gas sensor to remain compliant with regulatory time response standards (T20, T50, T90) for gas sensing. The design of the filter assembly also meets these standard response times without the use of a software accelerator.

In addition, since at least some process installations may involve highly volatile or even explosive environments, it is often beneficial, or even required, for devices that operate in such environments to comply with intrinsic safety requirements. These requirements help ensure that compliant electrical devices will not generate a source of ignition even under fault conditions. One example of an Intrinsic Safety requirement is set forth in: APPROVAL STANDARD INTRINSICALLY SAFE APPARATUS AND ASSOCIATED APPARATUS FOR USE IN CLASS I, II and III, DIVISION NUMBER 1 HAZARDOUS (CLASSIFIED) LOCATIONS, CLASS NUMBER 3610, promulgated by Factory Mutual Research October, 1998.

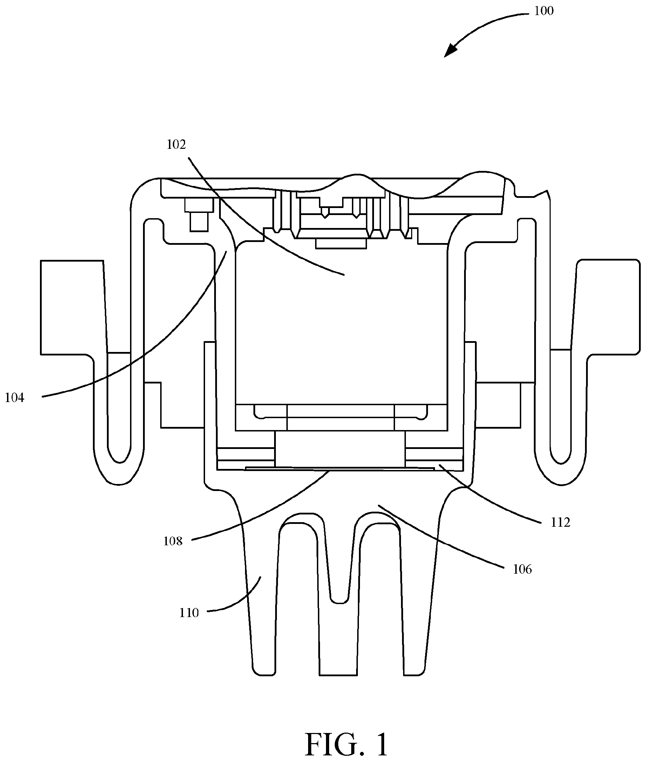

FIG. 1 shows a cross sectional view of a gas sensor module coupled to a filter assembly in accordance with an embodiment of the present invention. Gas detection device 100 includes a gas sensor module (e.g., generally a gas sensor 102 within a gas sensor housing 104) coupled to a filter assembly 106. Gas sensor 102 may be configured to detect combustible, toxic, and/or flammable gases present in a particular environment. Filter assembly 106, in one embodiment, is configured to protect gas sensor 102 from debris and moisture while still allowing gas sensor 102 to provide accurate readings in accordance with industry standard response times.

Filter assembly 106, in one embodiment, includes a filter housing 110. In one embodiment, upon coupling filter assembly 106 and gas sensor housing 104, filter housing 110 is configured to encircle a lower portion of gas sensor housing 104. Filter housing 110 may include any suitable design that allows gas sensor 102 to provide and maintain accurate readings in accordance with industry standards, while allowing for a protection against debris and moisture.

Filter assembly 106 may also include a gasket 112 and a filter element 108, each configured to be compressed against gas sensor housing 104 upon coupling filter assembly 106 to gas sensor housing 104. In one embodiment, filter element 108 is disposed at or near the center of filter assembly 106, and at least a portion of gasket 112 is placed on top of filter element 108. Upon being compressed, gasket 112 and filter element 108, in one embodiment, create a compression seal and, along with filter housing 110, provide IP66 rated protection for gas sensor 102. Filter element 108 may include any permeable material configured to allow for an exclusive passage of gas, thereby allowing gas sensor 102 to provide accurate readings in accordance with industry standard response times. In addition, filter element 108 may also include a hydrophobic, permeable material configured to allow for an exclusive passage of gas while inhibiting moisture from reaching gas sensor 102. Gasket 112 may include a hydrophobic material configured to inhibit moisture from reaching gas sensor 102.

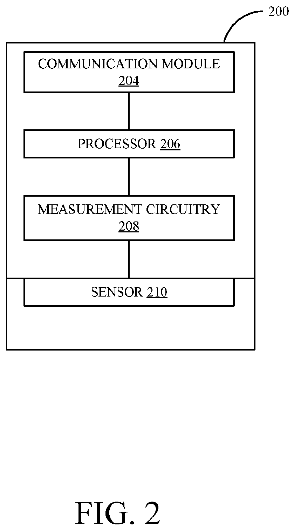

FIG. 2 is a diagrammatic system block diagram of a gas sensor module in accordance with an embodiment of the present invention. It is preferred that gas sensor module 200 comply with at least one intrinsic safety specification, such as that listed above, in order to help ensure safety in potentially explosive environments. Gas sensor module 200 includes a communication module 204 that is couplable to transmitter or other suitable device for conveying gas detection information to a control system, annunciation panel, or any other suitable device. This communication is in accordance with any suitable protocol. Communication module 204 is coupled to processor 206, which may be, in some embodiments, a microprocessor. Processor 206 is coupled to measurement circuitry 208 and is able to programmatically calculate one or more process variables, indicative of a gas, based on measurements of the raw sensor values obtained from measurement circuitry 208. Measurement circuitry 208, in one embodiment, is configured to receive signal information generated by sensor 210 and may include one or more suitable analog-to-digital converters as well as linearization circuitry and/or suitable filters, as appropriate.

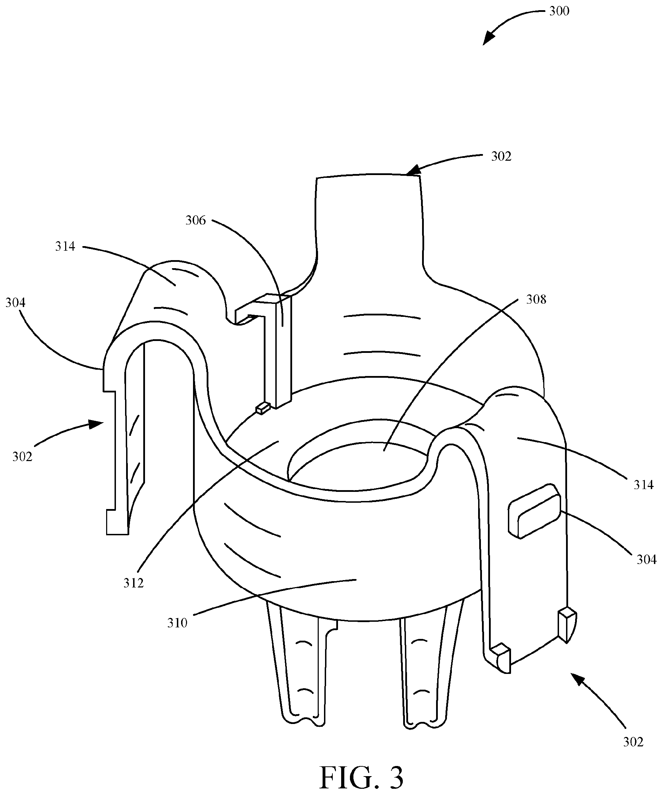

FIG. 3 is a diagrammatic view of a filter assembly that is field-replaceable in accordance with an embodiment of the present invention. Filter assembly 300, in one embodiment, is configured to protect a gas sensor (e.g. gas sensor 102 shown in FIG. 1) from moisture and debris in accordance with an Ingress Protection standard. In one embodiment, filter assembly 300 is configured to allow for an exclusive passage of gas, from an exterior environment, to a gas sensor, while inhibiting passage of moisture and/or debris. In one embodiment, filter assembly 300 is configured to be tool-lessly coupled to a gas sensor housing (e.g. gas sensor housing 104 shown in FIG. 1). Filter assembly 300, in one embodiment, includes a filter housing 310 with latching features 302 configured to couple filter assembly 300 to a gas sensor housing. In addition, filter housing 310 may include an alignment mechanism 306 configured to align filter assembly 300 with a gas sensor housing prior to the coupling of filter assembly 300 to the gas sensor housing.

Filter assembly 300 may include a filter element 308, disposed at or near the center of filter assembly 300. Filter assembly 300 may also include a gasket 312, such that at least a portion of gasket 312 is placed on top of filter element 308. In one embodiment, filter element 308 includes a permeable material configured to allow for a passage of gas. In one embodiment, filter element 308 includes a hydrophobic, permeable material. In one embodiment, upon coupling filter assembly 300 to a gas sensor housing using latching features 302, filter element 308 and gasket 312 become compressed, creating a seal between filter housing 310 and the gas sensor housing. In one embodiment, this compression provides an IP66 rated seal, as long as filter assembly 300 is coupled to the gas sensor housing, while still allowing a gas sensor to be responsive in accordance with known industry standards.

In one embodiment, latching features 302 include an inverted "U" shaped body 314 and a protrusion 304. Protrusion 304, in one embodiment, is configured to be received by a receiving portion in a gas sensor housing, and, upon being received, couple filter assembly 300 to a gas sensor housing. As illustratively shown, filter assembly 300 includes three latching features generally represented by reference numeral 302, however, in other embodiments, it is expressly contemplated that any suitable number of latching features may be used in accordance with an embodiment of the present invention.

In one embodiment, latching features 302 allow for a tool-less coupling of filter assembly 300 and a gas sensor housing. For example, but not by limitation, upon applying an insertion force, latching features 302 may latch to a receiving portion on a gas sensor housing, therein securing filter assembly 300 to the gas sensor housing without requiring any tools. In one embodiment, filter assembly 300 may then be removed by hand from the gas sensor housing by compressing latching features 302 and pulling filter assembly 300 from the gas sensor housing.

Prior to the coupling of filter assembly 300 to a gas sensor housing using one or more latching features 302, alignment mechanism 306 orients filter assembly 300 such that the one or more latching features 302 are in a proper orientation to the gas sensor housing. This ensures that filter assembly 300 may be installed without risk of damage to the gas sensor and/or the gas sensor housing. In the invention, alignment mechanism 306 includes a groove within a sidewall of filter housing 310, and is configured to align with, and axially receive, a corresponding alignment portion on a gas sensor housing. In one embodiment, the alignment between alignment mechanism 306 and a corresponding alignment portion ensures that filter assembly 300 is coupled to a gas sensor housing when in a proper rotational orientation. A proper rotational orientation, in one embodiment, is when the gas sensor housing and filter assembly 300 are properly oriented, with respect to one another. In one embodiment, the corresponding alignment portion may include an axial ridge configured to be axially received by alignment mechanism 306.

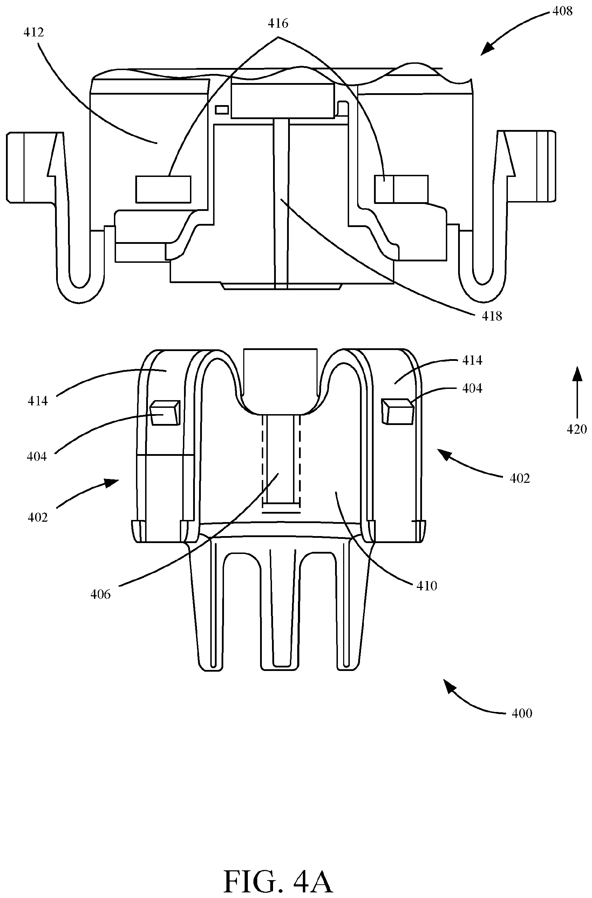

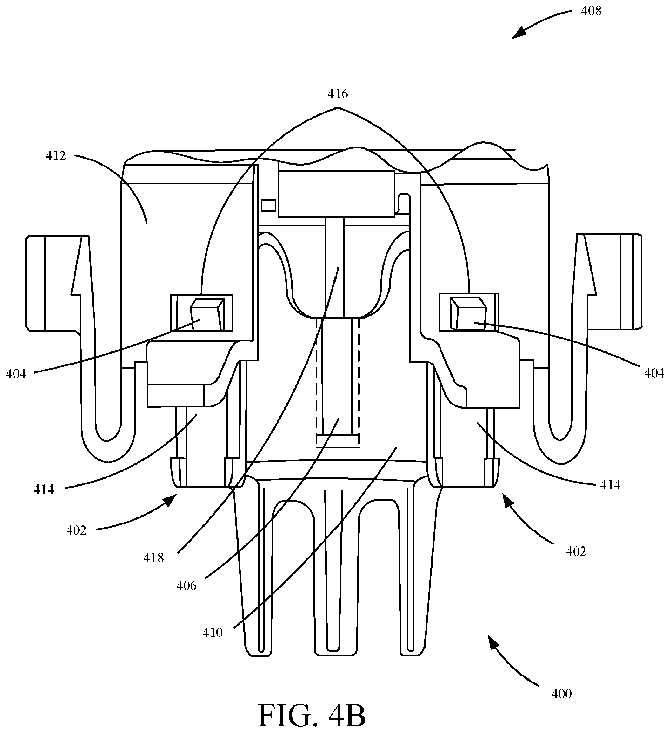

FIG. 4A and 4B are diagrammatic views of a filter assembly aligned with a gas sensor module in accordance with an embodiment of the present invention.

FIG. 4A illustrates a filter assembly 400 aligned with a gas sensor module 408. In one embodiment, gas sensor module 408 includes a gas sensor (not shown) within a gas sensor housing 412. In one embodiment, filter assembly 400 is coupled to gas sensor housing 412 using a straight-line insertion operation, as illustratively shown in FIGs. 4A and 4B, such that any potential damage to the gas sensor or gas sensor housing 412 which (typically occurs in conventional, threaded designs) is mitigated. The straight-line insertion operation, in one embodiment, includes aligning an alignment mechanism 406 of a filter housing 410 with a corresponding alignment portion 418 on gas sensor housing 412, and applying an insertion force in a direction generally indicated by arrow 420, such that latching features 402 of filter assembly 400 latch to receiving portions 416. For example, FIG. 4A shows aligning alignment mechanism 406 with corresponding alignment portion 418, and FIG. 4B shows filter assembly 400 coupled to gas sensor housing 412 after an insertion force is applied. As used herein, a straight-line insertion operation is intended to mean that the two components do not rotate relative to one another during the operation. This is advantageous as it prevents rotation that could damage the compression seal.

In the invention, latching features 402 include an inverted "U" shaped body 414 with a protrusion 404. Protrusions 404, in one embodiment, are configured to be received by receiving portions 416, respectively, of gas sensor module 408 upon applying an insertion force in the direction generally indicated by arrow 420. In the invention the alignment mechanism 406 is a groove configured to come into contact with corresponding alignment portion 418. In one embodiment, corresponding alignment portion 418 includes an axial ridge. In one embodiment, filter assembly 400 is tool-lessly coupled to gas sensor housing 412 using the straight-line insertion operation.

FIG. 4B illustrates a gas detection device after insertion force 420 is applied to filter assembly 400, such that filter assembly 400 is coupled to gas sensor module 408. As illustratively shown, protrusions 404, of latching features 402, are received by receiving portions 416, respectively, of gas sensor housing 412. Additionally, corresponding alignment portion 418, of gas sensor module 408, is received by alignment mechanism 406 of filter assembly 400. In one embodiment, filter assembly 400 may be removed from gas sensor module 408 by hand, without the use of tools, through the compression of latching features 402 and subsequent pulling of filter assembly 400 from gas sensor module 408.

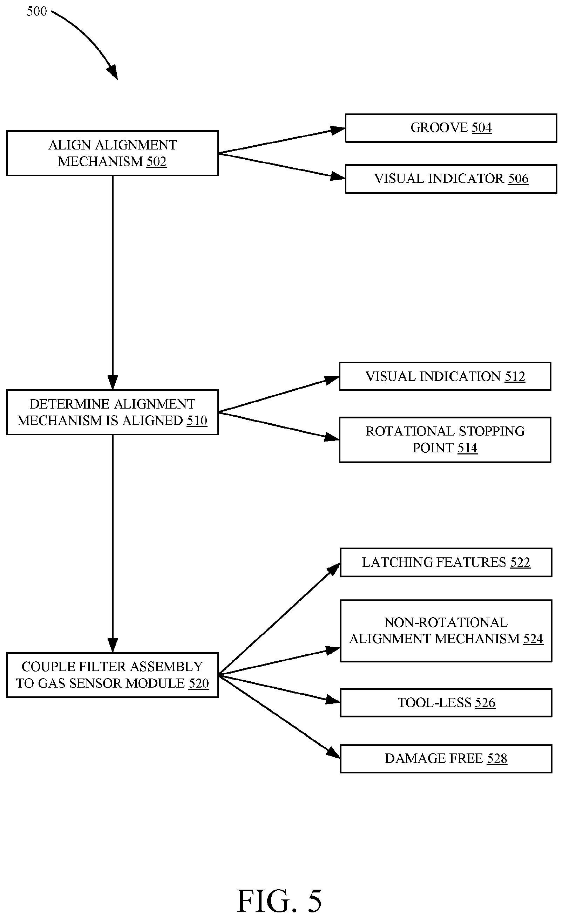

FIG. 5 is a flow diagram of installing a filter assembly to a gas sensor module in accordance with an embodiment of the present invention. Method 500 may be useful in installing a filter assembly without needing tools or having to thread a portion of a filter which may lead to damage of a gas sensor module. Method 500 begins at block 502, where a user aligns an alignment mechanism of a filter assembly with a corresponding alignment portion on a gas sensor module. While method 500 may be performed by a user, it is also expressly contemplated that in other embodiments, method 500 is performed automatically during manufacture. In one embodiment, the alignment mechanism includes a groove, as indicated at block 504, configured to receive the corresponding alignment portion on the gas sensor module. In one embodiment, the groove is specific to the corresponding alignment portion, such that the filter assembly is only coupled to the gas sensor module after aligning the alignment mechanism with the corresponding alignment portion. In one embodiment, this ensures that the filter assembly is in a correct rotational orientation prior to applying an insertion force. In one embodiment, the alignment mechanism includes a visual indicator, as indicated at block 506. In one embodiment, the visual indicator may include a color or a pattern on the filter assembly.

At block 510, a user determines that an alignment mechanism of a filter assembly is aligned with a corresponding alignment portion on a gas sensor module. In one embodiment, a determination may include a visual indication, as indicated at block 512. The visual indication may include an actuated light or a pattern that forms when the alignment mechanism is aligned with the corresponding alignment portion on the gas sensor module, for example. In another embodiment, a determination may include a rotational stopping point, as indicated at block 514. As the filter assembly is rotated relative to the gas sensor module, the rotational stopping point may cease rotational movement of the filter assembly once the alignment mechanism and the corresponding alignment portion are aligned. In addition, other detection mechanisms are envisioned in other embodiments.

At block 520, a user applies an insertion force to a filter assembly such that the filter assembly becomes coupled to a gas sensor module. In one embodiment, the insertion force is applied to the filter assembly until latching features of the filter assembly latch to a gas sensor housing of the gas sensor module, as indicated at block 522. In one embodiment, the filter assembly is securely fastened to the gas sensor module upon a mechanical latching of latching features to the gas sensor housing. In another embodiment, as the filter assembly is coupled to the gas sensor module, an alignment mechanism of the filter assembly receives a corresponding alignment portion of the gas sensor module, as indicated at block 524. In one embodiment, the insertion of the filter assembly into the gas sensor module is done without any tools, as indicated at block 526. In one embodiment, the insertion of the filter assembly into the gas sensor module in accordance with methods discussed herein, ensures that there is no damage done to the gas sensor, as indicated at block 528.

Thus, method 500 allows a user to install a filter assembly without having to follow a particular protocol requiring significant amounts of time or a variety of tools.

## Claims

1. A gas detection device (100) comprising:
a gas sensor module configured to detect a gas, comprising;
a gas sensor (102) disposed within a gas sensor housing (104);
circuitry (208) coupled to the gas sensor housing, the circuitry being configured to provide an indication of the gas; and
a filter assembly (106) configured to sealingly couple to the gas sensor housing via a straight line insertion operation and protect the gas sensor from particulates, comprising:
at least one latching feature (302) configured to couple to the gas sensor module, the at least one latching feature comprising an inverted U-shaped body (314) having a protrusion (304) configured to be received by a receiving portion on the gas sensor module
**characterised in that**:
the filter assembly comprises an alignment mechanism (306) configured to align with a corresponding alignment portion on the gas sensor housing, and wherein the alignment mechanism is a groove on the filter assembly configured to receive the corresponding alignment portion on the gas sensor housing.

2. The gas detection device of claim 1, wherein the filter assembly is configured to be tool-lessly coupled to the gas sensor housing.

3. The gas detection device of claim 1, wherein the gas detection device is intrinsically-safe.

4. The gas detection device of claim 1, wherein the filter assembly further comprises a gasket (312) and a filter element (308) configured to create a compression seal upon coupling the filter assembly to the gas sensor housing and allow for an exclusive passage of the gas.

5. The gas detection device of claim 4, wherein the filter element is hydrophobic.

6. The gas detection device of claim 4, wherein the compression seal is an ingress-protection rated seal.

7. The gas detection device of claim 1, wherein the gas sensor has a type, and wherein the filter assembly has an alignment mechanism that corresponds to the gas sensor type.

8. A method of installing the filter assembly (106) of claim 1 in the gas detection device (100) of claim 1, the method comprising:
aligning the alignment mechanism (306) of the filter assembly with a corresponding alignment portion of the gas sensor module;
determining that the alignment mechanism of the filter assembly is aligned with the corresponding alignment portion on the gas sensor housing;
applying an insertion force to the filter assembly such that the inverted U-shaped latching feature of the filter assembly engages a receiving portion on the gas sensor housing; and
wherein applying the insertion force couples the filter assembly to the gas sensor housing without using any tools.

9. The method of claim 8, wherein determining comprises detecting a visual indication.

10. The method of claim 9, wherein the visual indication is a pattern that forms when the alignment mechanism is aligned with the corresponding alignment portion of the housing device.

11. The method of claim 8, wherein determining comprises detecting a rotational stop.

## Patentansprüche

1. Gasdetektionsvorrichtung (100), umfassend:
ein Gassensormodul, das konfiguriert ist, um ein Gas zu erkennen, umfassend;
einen Gassensor (102), der innerhalb eines Gassensorgehäuses (104) angeordnet ist;
eine Schaltung (208), die mit dem Gassensorgehäuse gekoppelt ist, wobei die Schaltung konfiguriert ist, um eine Anzeige des Gases bereitzustellen; und
eine Filteranordnung (106), die konfiguriert ist, um mit dem Gassensorgehäuse über einen geradlinigen Einführvorgang dichtend zu koppeln und den Gassensor vor Partikeln zu schützen, umfassend:
mindestens ein Verriegelungsmerkmal (302), das konfiguriert ist, um mit dem Gassensormodul zu koppeln, wobei das mindestens eine Verriegelungsmerkmal einen umgekehrt U-förmigen Körper (314) mit einem Vorsprung (304) umfasst, der konfiguriert ist, um von einem Aufnahmeabschnitt am Gassensormodul aufgenommen zu werden,
**dadurch gekennzeichnet, dass**:
die Filteranordnung einen Ausrichtungsmechanismus (306) umfasst, der konfiguriert ist, um mit einem entsprechenden Ausrichtungsabschnitt am Gassensorgehäuse ausgerichtet zu sein, und wobei der Ausrichtungsmechanismus eine Nut an der Filteranordnung ist, die konfiguriert ist, um den entsprechenden Ausrichtungsabschnitt an dem Gassensorgehäuse aufzunehmen.

2. Gasdetektionsvorrichtung nach Anspruch 1, wobei die Filteranordnung konfiguriert ist, um ohne Werkzeuge mit dem Gassensorgehäuse gekoppelt zu werden.

3. Gasdetektionsvorrichtung nach Anspruch 1, wobei die Gasdetektionsvorrichtung eigensicher ist.

4. Gasdetektionsvorrichtung nach Anspruch 1, wobei die Filteranordnung ferner eine Dichtung (312) und ein Filterelement (308) umfasst, die konfiguriert sind, um eine Kompressionsdichtung beim Koppeln der Filteranordnung mit dem Gassensorgehäuse zu erzeugen und einen ausschließlichen Durchtritt des Gases zu ermöglichen.

5. Gasdetektionsvorrichtung nach Anspruch 4, wobei das Filterelement hydrophob ist.

6. Gasdetektionsvorrichtung nach Anspruch 4, wobei die Kompressionsdichtung eine Dichtung mit IP-Schutzklassifizierung ist.

7. Gasdetektionsvorrichtung nach Anspruch 1, wobei der Gassensor einen Typ aufweist, und wobei die Filteranordnung einen Ausrichtungsmechanismus aufweist, der dem Gassensortyp entspricht.

8. Verfahren zum Installieren der Filteranordnung (106) nach Anspruch 1 in der Gasdetektionsvorrichtung (100) nach Anspruch 1, wobei das Verfahren umfasst:
Ausrichten des Ausrichtungsmechanismus (306) der Filteranordnung mit einem entsprechenden Ausrichtungsabschnitt des Gassensormoduls;
Bestimmen, dass der Ausrichtungsmechanismus der Filteranordnung mit dem entsprechenden Ausrichtungsabschnitt an dem Gassensorgehäuse ausgerichtet ist;
Aufbringen einer Einführkraft auf die Filteranordnung, sodass das umgekehrt U-förmige Verriegelungsmerkmal der Filteranordnung mit einem Aufnahmeabschnitt an dem Gassensorgehäuse in Eingriff kommt; und
wobei das Aufbringen der Einführkraft die Filteranordnung mit dem Gassensorgehäuse koppelt, ohne Werkzeuge zu verwenden.

9. Verfahren nach Anspruch 8, wobei das Bestimmen das Erkennen einer visuellen Anzeige umfasst.

10. Verfahren nach Anspruch 9, wobei die visuelle Anzeige ein Muster ist, das entsteht, wenn der Ausrichtungsmechanismus mit dem entsprechenden Ausrichtungsabschnitt der Gehäusevorrichtung ausgerichtet ist.

11. Verfahren nach Anspruch 8, wobei das Bestimmen das Erkennen eines Drehanschlags umfasst.

## Revendications

1. Dispositif de détection de gaz (100) comprenant :
un module de capteur de gaz configuré pour détecter un gaz, comprenant ;
un capteur de gaz (102) disposé au sein d'un boîtier de capteur de gaz (104) ;
une circuiterie (208) couplée au boîtier de capteur de gaz, la circuiterie étant configurée pour fournir une indication du gaz ; et
un ensemble de filtre (106) configuré pour se coupler de manière étanche au boîtier de capteur de gaz par une opération d'insertion en ligne droite et protéger le capteur de gaz des particules, comprenant :
au moins une caractéristique de blocage (302) configurée pour se coupler au module de capteur de gaz, l'au moins une caractéristique de blocage comprenant un corps en forme de U inversé (314) ayant une saillie (304) configurée pour être reçue par une portion de réception sur le module de capteur de gaz
**caractérisé en ce que** :
l'ensemble de filtre comprend un mécanisme d'alignement (306) configuré pour s'aligner avec une portion d'alignement correspondante sur le boîtier de capteur de gaz, et dans lequel le mécanisme d'alignement est une rainure sur l'ensemble de filtre configurée pour recevoir la portion d'alignement correspondante sur le boîtier de capteur de gaz.

2. Dispositif de détection de gaz selon la revendication 1, dans lequel l'ensemble de filtre est configuré pour être couplé sans outil au boîtier de capteur de gaz.

3. Dispositif de détection de gaz selon la revendication 1, dans lequel le dispositif de détection de gaz est à sécurité intrinsèque.

4. Dispositif de détection de gaz selon la revendication 1, dans lequel l'ensemble de filtre comprend en outre un joint (312) et un élément de filtre (308) configuré pour créer un joint d'étanchéité par compression lors de l'accouplement de l'ensemble de filtre au boîtier du capteur de gaz et pour permettre un passage exclusif du gaz.

5. Dispositif de détection de gaz selon la revendication 4, dans lequel l'élément de filtre est hydrophobe.

6. Dispositif de détection de gaz selon la revendication 4, dans lequel le joint d'étanchéité par compression est un joint d'étanchéité classé selon un indice de protection contre la pénétration.

7. Dispositif de détection de gaz selon la revendication 1, dans lequel le capteur de gaz est d'un type, et dans lequel l'ensemble de filtre a un mécanisme d'alignement qui correspond au type de capteur de gaz.

8. Procédé d'installation de l'ensemble de filtre (106) selon la revendication 1 dans le dispositif de détection de gaz (100) selon la revendication 1, le procédé comprenant :
l'alignement du mécanisme d'alignement (306) de l'ensemble de filtre avec une portion d'alignement correspondante du module de capteur de gaz ;
la détermination que le mécanisme d'alignement de l'ensemble de filtre est aligné avec la portion d'alignement correspondante sur le boîtier du capteur de gaz ;
l'application d'une force d'insertion à l'ensemble de filtre de telle sorte que la caractéristique de blocage en forme de U inversé de l'ensemble de filtre s'emboîte dans une portion de réception sur le boîtier du capteur de gaz ; et
dans lequel l'application de la force d'insertion accouple l'ensemble de filtre au boîtier de capteur de gaz sans utiliser aucun outil.

9. Procédé selon la revendication 8, dans lequel la détermination comprend la détection d'une indication visuelle.

10. Procédé selon la revendication 9, dans lequel l'indication visuelle est un motif qui se forme lorsque le mécanisme d'alignement est aligné avec la portion d'alignement correspondante du dispositif boîtier.

11. Procédé selon la revendication 8, dans lequel la détermination comprend la détection d'un arrêt de rotation.
